# EUROPÄISCHE PATENTSCHRIFT

(11) **EP 0 395 592 B1**
(45) Veröffentlichungstag und Bekanntmachung des Hinweises auf die Patenterteilung: **08.11.1995**
(21) Anmeldenummer: 90810325.2
(22) Anmeldetag: 25.04.1990
(51) Int. Cl.: A61F 13/00

(54) **Kompresse zur Versorgung von Wunden**
Compress for the treatment of wounds
Compresse pour soigner les plaies

(30) Priorität: 27.04.1989 CH 1607/89
(43) Veröffentlichungstag der Anmeldung: 31.10.1990
(73) Patentinhaber: Flawa Schweizer Verbandstoff- und Wattefabriken AG, CH-9230 Flawil (CH)
(72) Erfinder: Gerhartl, Gerd, CH-9248 Bichwil (CH)
(74) Vertreter: Kulhavy, Sava, Dipl.-Ing.

(56) Entgegenhaltungen:
- WO-A-86/01378
- GB-A- 1 588 933
- US-A- 2 896 618
- US-A- 3 017 304

## Beschreibung

Die vorliegende Erfindung betrifft eine Kompresse zur Versorgung von Wunden, mit einer Depotschicht zur Speicherung des Wundsekretes, sowie ein Verfahren zur Herstellung derselben.

Ein Gegenstand ähnlicher Gattung ist in WO 86/01378 offenbart. Bei diesem Gegenstand handelt es sich um einen saugfähigen Körper, welcher vor allem bei weiblicher Inkontinenz anwendbar ist.

Der genannte Körper ist im wesentlichen rechteckförmig und er weist einen saugfähigen Kern auf. Dieser Kern ist als eine Lage aus einem Material ausgeführt, welches eine hohe Absorbtionsfähigkeit für eine Flüssigkeit aufweist. Deswegen kann dieser Kern auch als Depotschicht bezeichnet werden. In der Oberseite dieser Materiallage ist eine Schar von parallel zueinander verlaufenden Rillen ausgeführt, welche sich in der Längsrichtung des Körpers erstrecken. Nach einer Wärmebehandlung der Materiallage wird das Material des Kernes im Bereich der vorgesehenen Rillen durch Schneiden oder Fräsen entfernt. Die dabei entstandenen Schnittflächen im Bereich der Seitenwände und des Bodens der Rillen stellen offene Flächen dar, durch welche die Flüssigkeit in das Innere des saugfähigen Kernes leicht und schnell versickern kann.

Die Dicke des Kernes ist verhältnismässig klein, sodass diese Sauglage nur wenig Flüssigkeit in sich aufnehmen kann. Um ein grosses Volumen von einer Flüssigkeit aufnehmen zu können, was besonders bei weiblicher Inkontinenz wichtig ist, sind Anhäufungen aus einem saugfähigen Material in den Endbereichen des länglichen und gerillten Kernes angeordnet. Zumindest die Vorderseite des gerillten Kernes ist mit einer Schicht aus Papier überzogen, welche bis zum Boden der jeweiligen Rille gezogen und dort befestigt ist. Diese Papierschicht ist für die Flüssigkeit weniger durchlässig als das Material des Kernes und deswegen hilft die Papierschicht, die Flüssigkeit aus dem mittleren Bereich der Rillen herauszuführen und den saugfähigen Materialanhäufungen am Ende des Kernes zuzuführen.

Die Benützung der Materialanhäufungen in den Endbereichen des länglichen Kernes erhöht zwar das Aufnahmevolumen dieser vorbekannten Einlage, aber die Materialanhäufungen, welche vorne und hinten vom Körper der Benützerin abstehen, können als unangenehm empfunden werden.

Die erste Aufgabe der vorliegenden Erfindung ist, eine Kompresse anzugeben, welche verhältnismässig flach ist und welche trotzdem ein sehr grosses Volumen an Körpersekretion aufnehmen kann.

Diese Aufgabe wird bei der Kompresse der eingangs genannten Gattung erfindungsgemäss so gelöst, wie dies im kennzeichnenden Teil des Anspruchs 1 definiert ist.

In US-PS 2 896 618 ist ein Körper offenbart, welcher jenem Körper ähnelt, der in der genannten WO-A 86/01378 offenbart ist. Im Kern dieses Körpers, welcher aus einem saugfähigen Material ist und welcher allein zur Speicherung des Sekretes dient, sind parallel zueinander verlaufende Kanäle eingeprägt. Ueber diesen Kern ist ein Film gezogen, in welchem sich Reihen von Löchern befinden. Diese Löcherreihen verlaufen parallel zueinander und der Abstand zwischen zwei benachbarten Löcherreihen gleicht dem Abstand zwischen zwei benachbarten Kanälen im Saugkern.

Bei der Herstellung dieses vorbekannten Saugkörpers muss man sehr darauf achten, dass die Löcherreihen zwischen zwei benachbarte Hügelzügen zu liegen kommen, welche links und rechts den betreffenden Kanal begrenzen. Da die Abstände zwischen den Löcherreihen bzw. den Hügelzügen verhältnismässig klein sind, ist es bei der Zusammenführung der Bahn aus dem saugfähigen Kern mit der Bahn aus dem perforierten Film während der Herstellung eines solchen Saugkörpers recht schwierig, die zwei Bahnen in die richtige Lage zueinander zu bringen und diese Lage der Bahnen dann während der Herstellung weiterer Saugkörper auch einzuhalten.

Eine weitere Aufgabe der vorliegenden Erfindung ist somit, ein Verfahren anzugeben, welches es ermöglicht, die vorliegenden Kompressen problemlos herzustellen.

Diese Aufgabe wird beim Verfahren der eingangs genannten Gattung erfindungsgemäss so gelöst, wie dies im kennzeichnenden Teil des Anspruchs 1 definiert ist.

Nachstehend werden Ausführungsbeispiele der vorliegenden Erfindung anhand der beiliegenden Zeichnungen näher erläutert. Es zeigt:
Fig. 1 perspektivisch und teilweise im aufgerollten Zustand die vorliegende Kompresse,
Fig. 2 schematisch in einer Explosionsdarstellung die Kompresse gemäss Fig. 1, wobei diese Darstellung ermöglichen soll, die Funktionsweise der vorliegenden Kompresse zu veranschaulichen,
Fig. 3 in einer Draufsicht einen Ausschnitt aus einer weiteren Ausführungsform der vorliegenden Kompresse, bei welcher die Oberfläche der Kompresse mit einem netzartigen Abstandhalter versehen ist und
Fig. 4 in einer Draufsicht eine noch weitere Ausführungsform der vorliegenden Kompresse, bei welcher die Oberfläche der Kompresse mit Elektroden versehen ist.

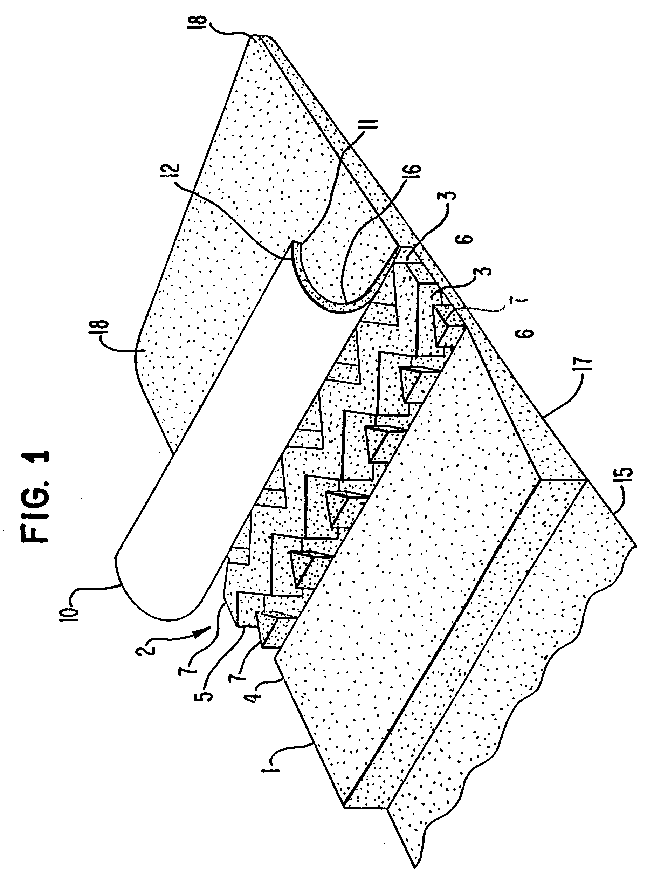

Die vorliegende Kompresse (Fig. 1) zur Versorgung von Wunden stellt ein dünnes und flächenhaftes Gebilde dar, welches beispielsweise viereckig sein kann. Die Kompresse kann etwa 3mm dick sein und die Grösse sowie die Form derselben kann sich nach dem jeweiligen Verwendungszweck richten. Die Kompresse weist aufeinander liegende Schichten 1, 2, 10 und 15 auf, welche untereinander verbunden sind, so dass die Kompresse ein Ganzes bildet. Die Ecken 18 der Kompresse sind abgerundet.

Die zuunterst liegende Schicht 15 der Kompresse ist im vorderen Bereich von Fig. 1 zur deutlicheren Darstellung dieser Schicht 15 verlängert gezeichnet, obwohl sie beim fertigen Produkt ebenfalls nahe am Rand der darauf liegenden Schicht 1 endet. Diese zuletzt genannte Schicht 1 besteht aus locker kardierter Watte, was durch einen keilförmigen Verlauf des aus dieser Zeichnung ersichtlichen, entlasteten Abschnittes dieser Schicht 1 angedeutet ist. Der linke Abschnitt der zuoberst liegenden Lage 10 ist teilweise aufgerollt, damit die darunter liegenden Schichten 1 und 2 ersichtlich sind. Die zwischen der obersten Lage 10 und der Watteschicht 1 liegende Schicht 2 ist gegenüber der Watteschicht 1 verkürzt dargestellt, damit ein Abschnitt der Watteschicht 1 von oben her ebenfalls ersichtlich ist.

Jene Seite der Kompresse, welche zur Auflage auf eine Wunde bestimmt ist, wird hier als Vorder- bzw. Wundseite der Kompresse bezeichnet. Die vorliegende Erfindung beruht auf einer Erkenntnis, wonach es für eine rasche Verteilung des Wundsekretes über die Fläche der Kompresse von entscheidender Bedeutung ist, wie die Schichtung der Kompresse ausgeführt ist und wie die Dichten in den einzelnen Schichten lokalisiert und verteilt sind.

Die dickste Schicht der vorliegenden Kompresse stellt eine sogenannte Depotschicht 1 dar, welche zur Speicherung des aus einer Wunde austretenden Sekretes dient. Da diese Schicht 1 ausserdem auch mechanische Einwirkungen auf die Wunde abfangen kann, kann diese Depotschicht 1 auch als ein Polster bzw. Kissen bezeichnet werden. Die Dicke der Depotschicht 1 wird beispielsweise zwischen 1mm und 2mm liegen. Das Kissen 1 erstreckt sich beinahe über die gesamte Fläche der Kompresse. Locker kardierte Watte bildet das Material dieser Depotschicht 1. Die Watte kann ausschliesslich aus Baumwolle bestehen oder sie kann ein Gemisch aus unterschiedlichen Fasern darstellen. Vorteilhaft besteht die Watte aus 80% Baumwoll- oder Viscose- und 20% Polyesterfasern.

Jene Seite der Depotschicht 1, welche der Wundseite der Kompresse zugewandt ist, ist mit einer sogenannten Steuerschicht 2 versehen. Diese Steuerschicht 2 ist derart ausgeführt, dass das Sekret, welches aus einer Wunde zur Kompresse gelangt, sich überwiegend entlang dieser Schicht 2 ausbreitet, bevor es zur Depotschicht 1 gelangt. Diese Schicht 2 steuert somit den Fluss des Sekretes in der Richtung der Fläche der Kompresse. Um diesen Effekt zu erreichen, sind Kanäle 3 in jener Oberfläche der Steuerschicht 2 ausgeführt, welche der Vorderseite der Kompresse zugewandt ist. Diese Kanäle 3 erstrecken sich in der Richtung der Längsabmessung der Steuerschicht 2 bzw. der Kompresse. Der Querschnitt dieser Kanäle 3 kann praktisch U- oder V-förmig sein.

Die Steuerschicht 2 ist aus oder sie enthält zumindest Fasern, welche vorteilhaft ein Vlies bilden. Es kann sich um ein Gemisch handeln, welches aus 80% Baumwoll- oder Viscose- und 20% Polyesterfasern besteht. Die zunächst lockeren Fasern bilden eine dicke Schicht, welche zusammengepresst (kalibriert) wird, damit eine verhältnismässig dünne, etwa 0,5mm bis 1mm dicke Schicht entsteht. In dieser Schicht 2 werden die Kanäle 3 durch eine örtliche und dauerhafte Verdichtung der Fasern (Prägung) in länglich verlaufenden Bereichen der Steuerschicht 2 ausgebildet. Die Dichte der Fasern im Bereich des Bodens 4 der Kanäle 3 ist somit grösser als die Dichte der Fasern im Bereich der Kanalwände 5. Die Kanäle 3 öffnen sich gegen die Vorderseite der Kompresse hin. Die obere Mündung des jeweiligen Kanals 3 ist durch die oberen Kanten 6 der Seitenwände 5 des Kanals 3 begrenzt und aus diesem Grund wird die Kanalmündung hier auch mit 6 bezeichnet. Die Kanalmündung 6 ist, wie dies aus dem soeben dargelegten hervorgeht, von der Depotschicht 1 abgewandt. Die Unterseite der den Kanalboden 4 bildenden Faserschicht steht in einem unmittelbaren Kontakt mit der Oberseite der Depotschicht 1. Dies ermöglicht einen Fluss des Sekretes aus der Steuerschicht 2 in die Depotschicht 1 hinein.

Im in Fig. 1 dargestellten Beispiel haben die Kanäle 3 einen sogenannten Zig-Zag-Verlauf und sie sind durch unverprägte und somit erhabene Abschnitte 7 des Materials der Steuerschicht 2 voneinander getrennt. Diese erhabenen Steuerschichtabschnitte 7 verlaufen logischerweise parallel zu den Kanälen 3, welche sie voneinander trennen. Von jenem der erhabenen und in Fig. 1 ersichtlichen Abschnitte 7, welcher von der aufgerollten Lage 10 entfernt liegt, sind nur Teile desselben mit dreieckförmigem Grundriss dargestellt. Diese Teile ergaben sich wegen dem in Fig. 1 geführten Schnitt.

Die in Fig. 1 dargestellten Kanäle 3 verlaufen im wesentlichen parallel zueinander und sie verlaufen quer zur grösseren Längsabmessung der Kompresse. Es versteht sich jedoch, dass die Kanäle 3 auch parallel zur grösseren Längsabmessung der Kompresse verlaufen können. Denkbar sind auch andere Formen von Kanälen 3. Die Kanäle 3 können sich beispielsweise kreuzen, sie können bestimmte Figuren bilden, usw. Die Tiefe der Kanäle 3 ist logischerweise kleiner als die Dicke der Steuerschicht 2. Die Dicke des Kanalbodens 4 kann der Tiefe des Kanals 3 gleichen oder sie kann kleiner als die Kanaltiefe sein.

Das Material der Steuerschicht 2 kann mit Wirksubstanzen wie mit Aktivkohle, blutstillenden Komponenten oder ähnlich ver setzt sein. Ausserdem ist es möglich, die Steuerschicht 2 durch eine Schicht aus einem Material zu ersetzen, welches Gel bildet, wenn es befeuchtet wird.

Die Vorderseite der Steuerschicht 2 ist mit einer Oberflächenlage 10 versehen, welche die Mündungen 6 in der Steuerschicht 2 überdeckt und welche den vorderen Abschluss der Kompresse bildet. Die Oberflächenlage 10 ist derart ausgeführt, dass sie für das aus der Wunde austretende Sekret durchlässig ist und dass sie mit der Wunde nicht verkleben kann. Sie kann beispielsweise durch eine perforierte Folie aus Aluminium gebildet sein, wenn die Kompresse zur Abdeckung von Brandwunden bestimmt ist. Die Oberflächenlage 10 kann jedoch auch aus Fasern sein, welche zumindest eine Schicht bilden.

Im dargestellten Beispiel weist die Oberflächenlage 10 zwei Schichten 11 und 12 aus Fasern auf, von welchen sich die erste Schicht 11 an der Aussenseite der Oberflächenlage 10 befindet. Die zweite Schicht 12 ist an jener Seite der Oberflächenlage 10 angeordnet, welche der Steuerschicht 2 zugewandt ist. Die Schichten 11 und 12 sind miteinander innig verbunden, so dass sie voneinander nicht getrennt werden können. Diese innige Verbindung der aus Fasern bestehenden Schichten kann beispielsweise durch Laminieren erreicht werden. Im Uebergangsbereich dieser Schichten ist ein Gemisch aus den Fasern der beiden Arten vorhanden. Die Fasern der Oberflächenlage 10 bilden vorteilhaft ein Vlies.

Die Flüssigkeit wird in der Oberflächenlage 10 nicht gespeichert. Dies deswegen, weil diese Lage 10 sehr dünn ist, weil das Material derselben durch das Wundsekret nicht benetzbar ist und weil das Wundsekret in den freien Raum der Kanäle 3 ungehindert eintreten kann. Die oberen Mündungen 6 der Kanäle 3 liegen nämlich an der rückwärtigen Seite der Oberflächenlage 10 auf. Hierbei helfen auch die Kapilarkräfte, das Wundsekret in die Kanäle 3 anzusaugen, weil die Breite der Kanäle sehr klein ist. Die Breite der Kanäle 3 kann zwischen 0,25mm und 1mm liegen. Ausserdem ragen noch einzelne Fasern des Materials der Steuerschicht 2 in den Hohlraum des jeweiligen Kanals 3 hinein, welche durch das Sekret gut benetzbar sind. Die Wundoberfläche bleibt in Folge dessen relativ trocken und die Wunde kann rasch heilen.

Die äussere Schicht 11, welche auch als Deckschicht bezeichnet werden kann, enthält vor allem Fasern aus einem Material, beispielsweise aus Polypropylen, welches für das Wundsekret abweisend ist. Die Dichte der Deckschicht 11 ist so gewählt, dass das Sekret, welches unter Umständen sogar zähflüssig sein kann, durch diese Schicht 11 passieren kann, ohne dass dem Durchgang des Sekretes durch die Deckschicht 11 ein nennenswerter Widerstand entgegengesetzt wird.

Die innere Schicht oder Bindeschicht 12 der Oberflächenlage 10 enthält vorwiegend Fasern aus einem Material, beispielsweise aus Polyäthylen, welches eine Verbindung zwischen den Fasern der Deckschicht 11 der Oberflächenlage 10 und den Fasern der Steuerschicht 2 ermöglicht. Die Bindeschicht 12 dient somit als ein Haftvermittler zwischen den Polypropylen-Fasern der Deckschicht und der kalibrierten (zusammengepressten) Watte der Steuerschicht 2.

Die Rückseite der Depotschicht 1 ist mit einer weitgehend flüssigkeitsdichten jedoch atmungsaktiven Schicht 15 versehen, welche die rückseitige Abschlusschicht der Kompresse darstellt. Diese Schicht 15, welche auch als Schutzschicht bezeichnet werden kann, ist als ein Mikrofaservlies, beispielsweise aus Polyäthylen, ausgeführt. Sie dient unter anderem als Wäscheschutz bei stark blutenden Wunden. Das Mikrofaservlies hat eine verhältnismässig rauhe Oberfläche, so dass die Rückseite dieser Schutzschicht auf jenem Abschnitt des Wäschestückes haften kann, welcher auf der Kompresse aufliegt. Dies Verhindert ein Verrutschen der Kompresse unter dem Wäschestück.

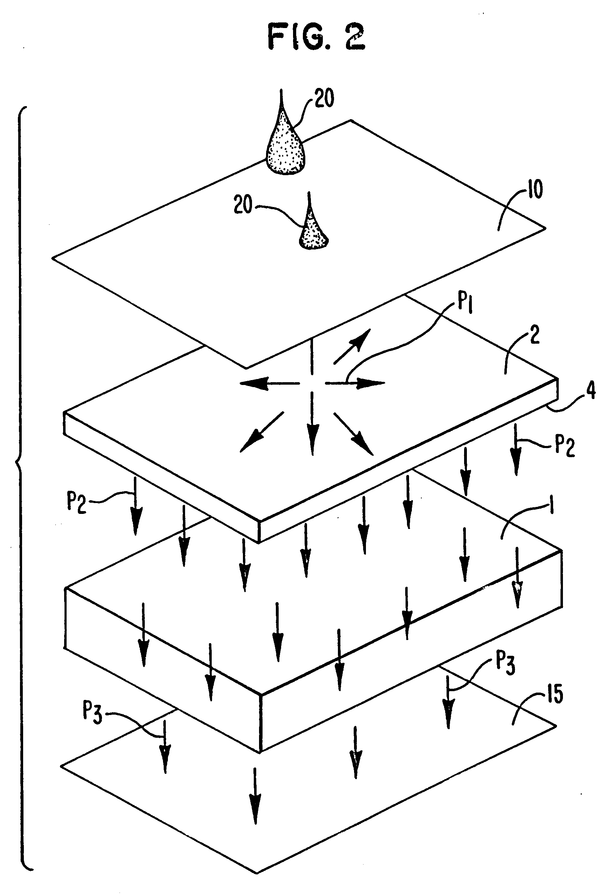

In Fig. 2 ist die vorliegende Kompresse schematisch und in einer Explosionsdarstellung gezeigt, damit die Funktionsweise der Kompresse veranschaulicht werden kann. Das Sekret, dargestellt durch Tropfen 20, dringt in die Wundseite der Kompresse hinein und hierbei passiert es zunächst die Oberflächenlage 10.

In Fig. 2 ist dies durch den ersten der dargestellten Tropfen 20 angedeutet, welcher teilweise nicht mehr ersichtlich ist. Wie dargelegt, passiert das Sekret die Oberflächenlage 10, ohne diese zu benetzen und gelangt zur Steuerschicht 2.

Durch die obere Kanalmündung 6 gelangt das Sekret in das Innere des Kanals 3 und füllt diesen in der Regel aus. Hierbei breitet sich das Sekret bis zum Kanalboden 4 aus. Da die Dichte des Fasermaterials im Bereich des Kanalbodens 4 verhältnismässig gross ist, kann das Sekret in den Kanalboden 4 nicht sofort eindringen. Die Dichte des Fasermaterials im Bereich der Kanalwände 5 ist zwar kleiner als die Dichte desselben im Bodenbereich 4, aber dafür ist der Weg länger, welchen das Sekret zurücklegen muss, wenn es aus dem Kanal 3 durch die Kanalwand 5 bis zur Depotschicht 1 gelangen soll. Dies verhindert, dass das Sekret, nachdem es den Kanal 3 gefüllt hat, auf diesem Wege in die Depotschicht 1 abfliesst.

Der Widerstand, welcher dem Fluss des Sekretes im Kanal 3 entgegengesetzt wird, ist wesentlich kleiner als jener Widerstand, welcher dem Einsickern des Sekretes in den Kanalboden 4 und in die Kanalwände 5 im Wege steht. Folglich fliesst das meiste Sekret senkrecht zur Eindringrichtung desselben durch den Kanal 3 zu jenen Stellen des Kanals 3, welche mit dem Sekret noch nicht gefüllt sind. Dies ist in Fig. 2 mit Hilfe der Pfeile P1 angedeutet. Da die Kanäle 3 sich in der Längsrichtung der Kompresse erstrecken, erfolgt in dieser Weise die bereits erwähnte rasche und wirksame Verteilung des Sekretes über die Fläche der Steuerschicht 2 und somit auch der Kompresse, bevor das Sekret in die Depotschicht 1 abfliesst.

Der Uebergang des Sekretes von der Steuerschicht 2 in die Depotschicht 1 vollzieht sich im Bereich der Berührungsstelle zwischen der Unterseite des Steuerschicht-Bodens 4 und der Oberseite der Depotschicht 1. Dieser Uebergang ist in Fig. 2 mit Hilfe der Pfeile P2 angedeutet. Diese Pfeile P2 sollen ausserdem andeuten, dass das Sekret sich von der praktisch zentral liegenden Eindringstelle der Kompresse bis in die Eckbereiche derselben dank der Steuerschicht 2 ausbreitet. Das Sekret kann durch die Depotschicht 1 bis zur Abschlusschicht 15 gelangen (Pfeile P3), wo das weitere Vordringen desselben allerdings verhindert wird, und zwar wegen den bereits beschriebenen Eigenschaften der Abschlusschicht 15.

Die Depotschicht 1 und die Steuerschicht 2 haben praktisch dieselben Abmessungen. Die Abmessungen der Oberflächenlage 10 und der unteren Abschlusschicht 15 sind ein wenig grösser als die Abmessungen des Kernes 1 und 2 der Kompresse, so dass die Oberflächenlage 10 und die Abschlusschicht 15 vom Kern 1, 2 abstehende Ränder 16 bzw. 17 aufweisen. Die Breite dieser abstehenden Randpartien 16 und 17 ist nur so gross gewählt, dass die Ränder 16 und 17 miteinander verschweisst werden können und dass der Kern 1, 2 sich in einer solchen Hülle 10, 15 nicht bewegen kann.

Bevor die einzelnen Lagen der vorliegenden Kompresse miteinander verbunden werden, werden diese Lagen getrennt vorbereitet. Die Steuerschicht 2 ergibt sich durch Kalibrieren einer Wattelage sowie durch Prägen einer solchen Schicht, wobei durch die Prägung die Kanäle 3 entstehen. Das Kalibrieren und Prägen kann unter Umständen in einem einzigen Arbeitsgang durchgeführt werden.

Die Oberflächenlage 10 wird durch Zusammenführen des Materials der Deckschicht 11 und jenes der Bindeschicht 12 sowie durch eine innige Verbindung dieser Lagen 11 und 12, beispielsweise durch Laminieren, hergestellt. Während des Herstellungsprozesses wird die Aussenseite der Deckschicht 11 gebügelt, woraus sich eine glatte, glänzende Oberfläche dieser Deckschicht 11 ergibt, welche eine der Ursachen dafür darstellt, dass die Oberflächenlage 10 nicht wundverklebend ist.

Das Material der Oberflächenlage 10 wird dann angewärmt und auf die Oberseite der Steuerschicht 2 gelegt, wo diese Oberflächenlage 10 abkühlen kann. Hierbei erfolgt eine Verklebung zwischen dem Material der Bindeschicht 12 und jenem der Steuerschicht 2. Anschliessend wird das Material der Depotschicht 1 jener Seite der Steuerschicht 2 zugeführt, welche der Rückseite der Kompresse zugewandt ist. Der Rückseite der Depotschicht wird das Material der Abschlusschicht 15 zugeordnet.

Vorteilhaft bilden die Materiale der einzelnen Lagen 1, 2, 10 und 15 Bahnen, welche während des Herstellungsprozesses zusammengeführt und in eine Einrichtung geführt werden, wo aus dieser Sandwich die einzelnen Kompressen herausgeschnitten werden. Dies kann beispielsweise unter der Anwendung der an sich bekannten Ultraschalltechnologie erfolgen.

Gleichzeitig mit dem Heraustrennen der Kompresse aus der Bahn erfolgt das Zusammenschweissen der Randpartien 16 und 17 der Oberflächenlage 10 und der Abschlusslage 15. Die bereits erwähnte Abrundung der Ecken 18 der Kompresse erfolgt ebenfalls während des Ausschneidens der Kompresse. Die Kompressen sind rundum weich verschlossen und es können keine Faserpartikeln aus diesen austreten.

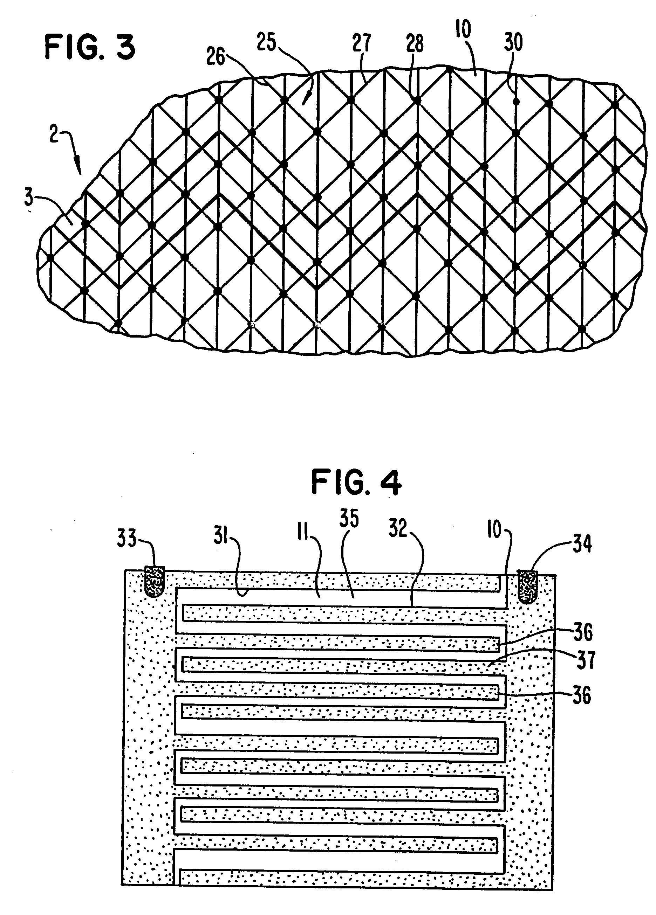

Fig. 3 zeigt in Draufsicht einen Ausschnitt aus einer weiteren Ausführungsform der vorliegenden Kompresse. Die einzelnen Bestandteile dieser Kompresse gleichen zumindest im wesentlichen den Bestandteilen jener Kompresse, welche z.B. im Zusammenhang mit Fig. 1 hier beschrieben worden ist.

Beträchtliche Probleme bei der Anwendung von Kompressen bzw. Wundkissen bereitet die Tatsache, dass die Oberfläche des auf eine Wunde aufgelegten Wundkissens mit den sich in der Wunde neu gebildeten Hautzellen verwächst. Beim Wechsel des Verbandes werden die mit dem Wundkissen verwachsenen Zellen von der Wunde weggerissen, wodurch der Zellbildungsprozess gestört und die Heilung der Wunde verzögert wird. Um diesen Nachteil zu beseitigen, befindet sich ein Abstandhalter 25 (Fig. 3) auf der Aussenseite der Oberflächenlage 10 der Kompresse. Der Abstandhalter 25 überdeckt wenigstens einen Teil der Aussenseite der Oberflächenlage 10 und er ist aus einem nicht benetzbaren Material. Vorteilhaft dient ein Metall, wie z.B. Silber oder Gold, als ein solches Material.

Die Atmung der Haut unter der Kompresse muss ausserdem so weitgehend wie möglich erleichtert werden. Deswegen ist der Abstandhalter 25 als ein netzähnliches Gebilde ausgeführt, durch dessen Maschen nicht nur Luft sondern auch Flüssigkeiten, insbesondere die Wundsekretion, hindurchgehen können. Da der Abstandhalter 25 nur einschichtig ist und da die Oberfläche desselben aus einem nicht benetzbaren Material ist, wird die Wundsekretion vom Abstandhalter 25 nicht aufgesaugt, sondern sie geht durch diesen hindurch und gelangt durch die Oberflächenlage 10 in den Körper der Kompresse, wo sie aufgenommen und gespeichert wird.

Der Abstandhalter bzw. das Netz 25 kann aus Metall, wie z.B. aus Silber oder Gold sein. Das Netz 25 kann jedoch auch so ausgeführt sein, dass der Kern desselben aus einem Kunststoffe wie z.B. aus Polyester, Polyamid oder Polypropylen ist und dass die Oberfläche dieses Kernes mit einem Metall beschichtet ist. Da Gold und Silber verhältnismässig teuere Materiale sind, kann der in dieser Weise ausgeführte Abstandhalter 25 kostengünstiger hergestellt werden als der rein metallische Abstandhalter. Zudem kann die Zugfestigkeit des nur metallisierten Abstandhalters 25, dank den für diesen Anwendungsfall günstigen Eigenschaften von Kunststoff, sogar noch grösser sein als die Zugfestigkeit eines Abstandhalters aus Metall.

Die Form und die Grösse der Maschen des netzförmigen Abstandhalters 25 sind so gewählt, dass die Wundsekretion möglichst ungehindert den Abstandhalter passieren kann. In Fig. 3 ist ein für den vorliegenden Fall zweckmässig ausgebildetes Netz 25 dargestellt, welches auf der Oberflächenschicht 10 aufgetragen und mit dieser fest verbunden ist. Unter der Oberflächenschicht 10 ist in Fig. 3 ein Abschnitt eines der Kanäle 3 in der Steuerschicht 2 der vorliegenden Kompresse ersichtlich.

Das Netz 25 weist zwei Scharen 26 und 27 von Streifen auf, welche auf der Aussenseite, d.h. auf der Deckschicht 11 der Oberflächenlage 10 aufliegen. Diese Streifen 26 und 27 liegen praktisch in derselben Ebene und sie kreuzen sich unter dem Einschluss eines spitzen Winkels zwischen sich. Im Bereich der Kreuzungsstellen dieser Streifenscharen 26 und 27 befinden sich flächenhafte Materialanhäufungen 28, mit deren Hilfe die jeweilige Kreuzungsstelle zweier Streifen 26 und 27 verstärkt ist. Die Materialanhäufungen 28 weisen eine kreisrunde Kontur auf und der Durchmesser dieser Anhäufungen 28 ist etwa zweimal so gross wie die Breite der Streifen 26 bzw. 27. Die sich kreuzenden Streifen 26 und 27 können sich wegen der Verbindung im Bereich der Kreuzungsstellen zueinander nicht verschieben.

Ferner umfasst das Netz 25 eine Schar von Stabilisierungstreifen 30, deren Breite der Breite der Diagonalstreifen 26 bzw. 27 vorteilhaft gleicht. Die Stabilisierungsstreifen 30 liegen in derselben Ebene wie die Diagonalstreifen 26 und 27 und die Materialanhäufungen 28. Der jeweilige Stabilisierungsstreifen 30 geht durch die Kreuzungsstellen 28 hindurch, welche in der Längsrichtung desselben hintereinander liegen. Man kann auch sagen, dass der jeweilige Abschnitt des Stabilisierungsstreifens 30, welcher sich zwischen zwei Kreuzungsstellen 28 erstreckt, gleich wie die entsprechenden Abschnitte der Diagonalstreifen 26 und 27 an die Materialanhäufungen 28 angeschlossen ist.

Das Vorhandensein der Stabilisierungsstreifen 30 bewirkt, dass das Netz 25 in der Richtung der Verbindungsstreifen 30 undehnbar ist, während das Netz in der dazu senkrechten Richtung dehnbar ist. Wenn es als zweckmässig erscheint, dann kann das Netz 25 noch eine zweite Schar von Stabilisierungsstreifen (nicht dargestellt) aufweisen, welche zu den Stabilisierungsstreifen 30 der ersten Schar senkrecht verlaufen bzw. stehen. Ein solches Netz ist undehnbar in den beiden zueinander senkrechten Richtungen.

Fig. 4 zeigt in einer Draufsicht eine noch weitere Ausführungsform der vorliegenden Kompresse. Es ist bekannt, dass der Durchfluss von elektrischem Strom, insbesondere eines Impulsstromes oder eines geringen konstanten Stromes durch eine Wundstelle eine intensivere Durchblutung dieser Stelle verursacht. Damit ist ein erhöhter Stoffwechsel an der so behandelten Wundstelle und somit auch eine bessere Versorgung dieser Wundstelle mit körpereigenen Abwehrstoffen verbunden.

Um diese Effekte bei der vorliegenden Kompresse erreichen zu können, ist die Aussenseite der Oberflächenlage 10, d.h. die Deckschicht 11 der Kompresse, mit Elektroden 31 und 32 versehen, welche auf der Oberflächenlage 10 haften. Im Prinzip reichen zwei einfache Elektroden aus, um den genannten Effekt erreichen zu können. Für spezielle Zwecke können jedoch auch mehrfache und/oder gegliederte Elektroden angewendet werden.

Den Elektroden 31 und 32 sind Anschlussfahnen 33 und 34 zugeordnet, welche mit den Elektroden 31 und 32 in elektrischem Kontakt stehen und an welchen die zu einer Stromquelle (nicht dargestellt) führenden Drähte angeschlossen sein können. Im dargestellten Beispiel sind die Anschlussfahnen 33 und 34 als U-förmige Klammern ausgeführt, welche auf einem Rand der Kompresse aufgesetzt sind. Zwischen den Schenkeln dieser Klammern 33 und 34 ist die Randpartie des textilen Teiles des Kompressenkörpers geklemmt, so dass die Klammern 33 und 34 auf diesem Teil des Kompressenkörpers festsitzen. Die Elektrode, welcher die jeweilige Klammer zugeordnet ist, kann zwischen einem der Klammerschenkel und dem textilen Teil der Kompresse geklemmt sein. Es versteht sich, dass die Anschlussfahnen 33 und 34 auch in einer anderen zweckmässigen Weise ausgeführt sein können und dass die Elektroden 31 und 32, je nach der Art und Weise der Ausführung derselben, an die Fahnen 33 und 34 auch in einer anderen Weise angeschlossen sein können.

Im einfachsten Fall können die Elektroden 31 und 32 als zwei in einem Abstand voneinander angeordnete Leiterbahnen ausgeführt sein, welche auf der Aussenseite der Oberflächenlage 10 angebracht sind. Wenn das Material des textilen Teiles des Kompressenkörpers, welches sich im Spalt 35 zwischen zwei benachbarten Elektroden 31 und 32 befindet, befeuchtet ist, beispielsweise durch die Wundsekretion, und wenn elektrische Spannung an die Elektroden 31 und 32 angelegt wird, dann fliesst elektrischer Strom zwischen den Leiterbahnen 31 und 32. Dieser Strom fliesst nicht nur durch die Oberflächenschicht 10 sondern auch durch die Wunde und dieser letztgenannte Strom wirkt sich in der heilenden Weise auf die Wunde aus.

In Fig. 4 sind Elektroden 31 und 32 dargestellt, von welchen jede annähernd die Form eines Kammes aufweist. Der Abstand zwischen den Zähnen 36 eines der Kämme 31 ist grösser als die Breite eines Zahnes 37 des anderen Kammes 32. Dies ermöglicht, dass die Zähne 36 eines der Kämme 31 zwischen den Zähnen 37 des anderen Kammes 32 angeordnet sein können. Bei einer solchen Ausführung und Anordnung der Elektroden 31 und 32 weist der Spalt 35 zwischen den Elektroden 31 und 32 eine sehr grosse Länge auf. Quer durch einen so langen Spalt 35 kann wesentlich mehr Strom fliessen als durch einen Spalt zwischen zwei bloss geradlinig verlaufende Elektroden 31 und 32. Ausserdem kann auch die Fläche der Wunde durch die Gliederung der Elektroden 31 und 32 wesentlich vergrössert werden, welche durch elektrischen Strom behandelt werden kann. In der Praxis kann es von Vorteil sein, die Elektroden spiralförmig, kreisförmig, ovalförmig oder anders auszuführen, die Kanten der Elektroden durch Anformung kleinerer Zähnen noch weiter zu gliedern usw.

## Patentansprüche

1. Kompresse zur Versorgung von Wunden, mit einer Depotschicht (1) zur Speicherung des Wundsekretes, dadurch gekennzeichnet, dass jener Seite der Depotschicht (1), welche der Vorderseite der Kompresse zugewandt ist, eine Steuerschicht (2) zugeordnet bzw. vorgeschaltet ist, dass diese Steuerschicht dünner ist als die Depotschicht, dass in jener Oberfläche der Steuerschicht (2), welche von der Depotschicht (1) abgewandt ist, Kanäle (3) ausgeführt sind, dass die Mündung (6) dieser Kanäle (3) von der Depotschicht (1) abgewandt ist und dass die Kanäle (3) sich in der Flächenrichtung der Steuerschicht (2) erstrecken, wobei sie ermöglichen, dass das Sekret, welches zur Steuerschicht (2) gelangt, sich überwiegend entlang dieser Schicht (2) ausbreiten kann, bevor es zur Depotschicht (1) gelangt.

2. Kompresse nach Anspruch 1, dadurch gekennzeichnet, dass die Steuerschicht (2) als eine Schicht aus zusammengepressten Fasern ausgeführt ist, dass die Kanäle (3) als örtliche Verdichtungen der Fasern ausgeführt sind, sodass die Dichte der Fasern im Bereich des Bodens (4) des Kanals (3) grösser ist als die Dichte der Fasern im Bereich der Kanalwände (5), dass die Dicke des Kanalbodens der Tiefe des Kanals (3) gleichen oder kleiner als diese sein kann und dass die Unterseite der den Kanalboden bildenden Faserschicht mit der Oberseite der Depotschicht (1) in Kontakt steht.

3. Kompresse nach Anspruch 1, dadurch gekennzeichnet, dass die Kanäle (3) sich gegen die Vorderseite der Kompresse hin öffnen und von der Depotschicht somit abgewandt sind und dass die Kanäle (3) einen praktisch U- oder V-förmigen Querschnitt aufweisen, wobei die Kanäle (3) im wesentlichen parallel zueinander liegen und einen Zig-Zag-förmigen Verlauf aufweisen können.

4. Kompresse nach Anspruch 1, dadurch gekennzeichnet, dass die Vorderseite der Steuerschicht (2) mit einer Oberflächenlage (10) versehen ist und dass diese Oberflächenlage (10) derart ausgeführt ist, dass sie für das Sekret durchlässig ist und dass sie mit der Wunde nicht verkleben kann.

5. Kompresse nach Anspruch 4, dadurch gekennzeichnet, dass die Oberflächenlage (10) aus zwei Schichten (11,12) besteht, welche für das Sekret durchlässig sind und welche miteinander innig verbunden sind, dass die äussere Schicht bzw. Deckschicht (11) vor allem ein Material, beispielsweise Polypropylen, enthält, welches für das Wundsekret abweisend ist, dass die innere Schicht bzw. Bindeschicht (12) vorwiegend ein Material, beispielsweise Polyäthylen, enthält, welches eine Verbindung zwischen der Deckschicht (11) und der Steuerschicht (2) ermöglicht, und dass die Oberflächenlage (10) Fasern enthalten kann, welche ein Vlies bilden.

6. Kompresse nach Anspruch 4, dadurch gekennzeichnet, dass die Oberflächenlage (10) als eine Membrane aus perforierter Aluminiumfolie ausgeführt ist.

7. Kompresse nach Anspruch 1, dadurch gekennzeichnet, dass die Rückseite der Depotschicht (1) mit einer weitgehend flüssigkeitsdichten jedoch atmungsaktiven Schicht (15) versehen ist und dass diese Schutzschicht (15) als ein Mikrofaservlies, beispielsweise aus Polyäthylen, ausgeführt sein kann.

8. Kompresse nach Anspruch 7, dadurch gekennzeichnet, dass die Depotschicht (1) und die Steuerschicht (2) praktisch dieselben Abmessungen haben, dass die Abmessungen der Oberflächenlage (10) und der unteren Abschlusschicht (15) ein wenig grösser sind als die Abmessungen des Kernes (1·2) der Kompresse, so dass die Oberflächenlage (10) und die Abschlusschicht (15) vom Kern (1,2) abstehende Ränder (16,17) aufweisen, dass die Breite dieser abstehenden Randpartien (16,17) nur so gross ist, dass die Ränder (16,17) miteinander verschweisst werden können und dass der Kern (1,2) sich in einer solchen Hülle (10,15) nicht bewegen kann.

9. Kompresse nach Anspruch 4, dadurch gekennzeichnet, dass die Aussenseite (11) der Oberflächenlage (10) mit einer Schicht (25) versehen ist, welche für das Wundsekret durchlässig ist und welche als ein Abstandhalter zwischen der Oberflächenlage (10) und der Wunde dient und dass der Abstandhalter (25) wenigstens einen Teil der Aussenseite der Oberflächenlage (10) überdeckt.

10. Kompresse nach Anspruch 9, dadurch gekennzeichnet, dass der Abstandhalter (25) als ein netzähnliches Gebilde ausgeführt ist, bei welchem wenigstens die Oberfläche desselben aus einem nicht benetzbaren Material, vorteilhaft aus Metall, wie z.B. aus Silber oder Gold, ist.

11. Kompresse nach Anspruch 10, dadurch gekennzeichnet, dass das Netz (25) aus Metall ist oder dass das Netz einen Kern aus einem Kunststoff, wie z.B. aus Polyester,Polyamid oder Polypropylen aufweist, dessen Oberfläche metallisiert ist.

12. Kompresse nach Anspruch 4, dadurch gekennzeichnet, dass das Netz (25) zwei Scharen (26,27) von Streifen aufweist, welche auf der Aussenseite der Oberflächenlage (10) aufliegen, dass diese Streifen (26,27) praktisch in derselben Ebene liegen und sich unter dem Einschluss eines spitzen Winkels zwischen sich kreuzen, dass im Bereich der Kreuzungsstellen dieser Streifenscharen (26,27) sich flächenhafte Materialanhäufungen (28) befinden, mit deren Hilfe die jeweilige Kreuzungsstelle zweier Streifen 26 und 27 verstärkt ist und dass die Materialanhäufungen (28) eine kreisrunde Kontur aufweisen und einen Durchmesser haben können, welcher etwa zweimal so gross ist wie die Breite der Streifen (26,27).

13. Kompresse nach Anspruch 12, dadurch gekennzeichnet, dass das Netz (25) zumindest eine Schar von Stabilisierungstreifen (30) umfasst, deren Breite der Breite der Diagonalstreifen (26, 27) vorteilhaft gleicht, dass die Stabilisierungsstreifen (30) in derselben Ebene liegen wie die Diagonalstreifen (26,27) und die Materialanhäufungen (28), dass der jeweilige Stabilisierungsstreifen (30) durch die Kreuzungsstellen (28) hindurchgeht, welche in der Längsrichtung desselben hintereinander liegen.

14. Kompresse nach Anspruch 13, dadurch gekennzeichnet, dass eine zweite Schar von Stabilisierungsstreifen vorgesehen ist, welche zu den Stabilisierungsstreifen (30) der ersten Schar senkrecht verlaufen bzw. stehen.

15. Kompresse nach Anspruch 4, dadurch gekennzeichnet, dass die Aussenseite (11) der Oberflächenlage (10) mit zumindest zwei nebeneinander liegenden Leiterbahnen bzw. Elektroden (31,32) versehen ist, welche an eine Stromquelle anschliessbar sind und dass diese Elektroden mehrfache und/oder gegliederte Elektroden sein können.

16. Kompresse nach Anspruch 15, dadurch gekennzeichnet, dass den Elektroden (31,32) Anschlussfahnen (33,34) zugeordnet sind, welche mit den Elektroden (31,32) in elektrischem Kontakt stehen und an welchen die zu einer Stromquelle führenden Drähte angeschlossen sein können.

17. Kompresse nach Anspruch 16, dadurch gekennzeichnet, dass die Anschlussfahnen (33,34) als U-förmige Klammern ausgeführt sind, welche auf einem Rand der Kompresse aufgesetzt sind, dass zwischen den Schenkeln dieser Klammern (33,34) die Randpartie des textilen Teiles des Kompressenkörpers geklemmt ist, und dass Elektrode, welcher die jeweilige Klammer zugeordnet ist, zwischen einem der Klammerschenkel und dem textilen Teil der Kompresse geklemmt ist.

18. Kompresse nach Anspruch 15, dadurch gekennzeichnet, dass die jeweilige Elektrode (31 bzw. 32) die Form eines Kammes aufweist, dass der Abstand zwischen den Zähnen (36) eines der Kämme (31) grösser ist als die Breite eines Zahnes (37) des anderen Kammes (32), und dass die Kanten der Elektroden durch Anformung kleinerer Zähne weiter gegliedert sein können.

19. Kompresse nach Anspruch 1, dadurch gekennzeichnet, dass das Material der Steuerschicht (2) mit Wirksubstanzen wie mit Aktivkohle, blutstillenden Komponenten oder ähnlich versetzt ist.

20. Kompresse nach Anspruch 2, dadurch gekennzeichnet, dass die Steuerschicht (2) durch eine Schicht aus einem Material gebildet ist, welches Gel bildet, wenn es befeuchtet wird.

21. Verfahren zur Herstellung der Kompresse nach Anspruch 1, dadurch gekennzeichnet, dass eine Materialschicht, aus welcher die Steuerschicht (2) entstehen soll, beispielsweise durch Prägen derart bearbeitet wird, dass sie Kanäle (3) aufweist, welche sich in der Flächenrichtung dieser Schicht (2) erstrecken, dass eine Oberflächenlage (10) angewärmt und auf die Oberseite der Steuerschicht (2) gelegt wird, wo sie abkühlen und sich dabei mit der Steuerschicht verbinden kann, dass eine Materialschicht, welche die Depotschicht (1) darstellt, jener Seite der Steuerschicht (2) zugeordnet wird, welche der Rückseite der Kompresse zugekehrt ist, und dass diese zwei Schichten (1 und 2) miteinander verbunden werden.

22. Verfahren nach Anspruch 21, dadurch gekennzeichnet, dass eine Schicht (15) aus einem weitgehend flüssigkeitsdichten jedoch atmungsaktiven Material jener Seite der Depotschicht (1) zugeordnet wird, welche der Rückseite der Kompresse zugewandt ist, und dass die Ränder (16,17) der Oberflächenlage (10) und der unteren Abschlusslage (15) miteinander, beispielsweise mit Hilfe von Ultraschall, verschweisst werden.

## Claims

1. Compress for dressing wounds, having a depot layer (1) for storing the secretion from the wound, characterized in that a guiding layer (2) is assigned to or superposed on that side of the depot layer (1) which faces the front side of the compress, in that said guiding layer is thinner than the depot layer, in that channels (3) are constructed in that surface of the guiding layer (2) which faces away from the depot layer (1), in that the opening (6) of these channels (3) faces away from the depot layer (1) and in that the channels (3) extend in the direction of the surface of the guiding layer (2), whereby they enable the secretion which reaches the guiding layer (2) to spread out predominantly along said layer (2), before it reaches the depot layer (1).

2. Compress according to Claim 1, characterized in that the guiding layer (2) is constructed as a layer of fibres pressed together, in that the channels (3) are constructed as local compressions of the fibres so that the density of the fibres in the region of the base (4) of the channel (3) is greater than the density of the fibres in the region of the channel walls (5), in that the thickness of the base of the channel can be equal to the depth of the channel (3) or smaller than the latter and in that the underside of the fibre layer forming the base of the channel is in contact with the top side of the depot layer (1).

3. Compress according to Claim 1, characterized in that the channels (3) open towards the front side of the compress and therefore face away from the depot layer and in that the channels (3) have a virtually U- or V-shaped cross-section, whereby the channels (3) are substantially parallel to one another and can have a zig-zag course.

4. Compress according to Claim 1, characterized in that the front side of the guiding layer (2) is provided with a surface ply (10) and in that said surface ply (10) is constructed in such a manner that it is permeable for the secretion and cannot adhere to the wound.

5. Compress according to Claim 4, characterized in that the surface ply (10) consists of two layers (11, 12) which are permeable for the secretion and which are intimately connected to one another, in that the outer layer or top layer (11) contains in particular a material, for example polypropylene, which repels the secretion from the wound, in that the inner layer or binder layer (12) contains predominantly a material, for example polyethylene, which enables the top layer (11) and the guiding layer (2) to be joined and in that the surface ply (10) can contain fibres which form a nonwoven.

6. Compress according to Claim 4, characterized in that the surface ply (10) is constructed as a membrane made of perforated aluminium foil.

7. Compress according to Claim 1, characterized in that the back of the depot layer (1) is provided with a layer (15) which is substantially liquid-tight but is capable of breathing, and in that said protective layer (15) may be constructed as a micro-nonwoven, for example of polyethylene.

8. Compress according to Claim 7, characterized in that the depot layer (1) and the guiding layer (2) have virtually the same dimensions, in that the dimensions of the surface ply (10) and of the lower sealing ply (15) are slightly larger than the dimensions of the core (1, 2) of the compress so that the surface ply (10) and the sealing ply (15) have edges (16, 17) which are at a distance from the core (1, 2) and in that the width of these remote edge sections (16, 17) is just large enough that the edges (16, 17) can be bonded together and that the core (1, 2) cannot move in an enclosure (10, 15) of this type.

9. Compress according to Claim 4, characterized in that the outer side (11) of the surface ply (10) is provided with a layer (25) which is permeable for the wound secretion and which serves as a spacer between the surface ply (10) and the wound and in that the spacer (25) overlaps at least part of the outer side of the surface ply (10).

10. Compress according to Claim 9, characterized in that the spacer (25) is constructed as a net-like formation, in which at least the surface thereof is made of a non-wettable material, advantageously metal, such as silver or gold for example.

11. Compress according to Claim 10, characterized in that the netting (25) is made of metal or in that the netting has a core made of a plastic such as polyester, polyamide or polypropylene for example, the surface of which is metallized.

12. Compress according to Claim 4, characterized in that the netting (25) has two systems (26, 27) of strips which are supported on the outer side of the surface ply (10), in that said strips (26, 27) are virtually in the same plane and intersect with inclusion of an acute angle between one another, in that in the region of the points of intersection of said systems of strips (26, 27) are located two-dimensional accumulations of material (28), with the aid of which the respective point of intersection of two strips 26 and 27 is reinforced and in that the accumulations of material (28) have a circular contour and can have a diameter which is approximately twice as large as the width of the strips (26, 27).

13. Compress according to Claim 12, characterized in that the netting (25) comprises at least one system of stabilizing strips (30), which advantageously have the same width as the diagonal strips (26, 27), in that the stabilizing strips (30) are in the same plane as the diagonal strips (26, 27) and the accumulations of material (28) and in that the respective stabilizing strip (30) passes through the points of intersection (28) which lie behind one another in the longitudinal direction of said strip.

14. Compress according to Claim 13, characterized in that a second system of stabilizing strips is provided which run or stand perpendicular to the stabilizing strips (30) of the first system.

15. Compress according to Claim 4, characterized in that the outer side (11) of the surface ply (10) is provided with at least two adjacent strip conductors or electrodes (31, 32) which may be connected to a source of electric power and in that said electrodes may be multiple and/or divided electrodes.

16. Compress according to Claim 15, characterized in that terminal lugs (33, 34) are assigned to the electrodes (31, 32), which terminal lugs are in electrical contact with the electrodes (31, 32) and to which the wires leading to a source of electrical power may be connected.

17. Compress according to Claim 16, characterized in that the terminal lugs (33, 34) are constructed as U-shaped brackets which are placed on one edge of the compress, in that the edge section of the textile part of the body of the compress is clamped between the legs of said brackets (33, 34) and in that the electrode to which the particular bracket is assigned is clamped between one of the bracket legs and the textile part of the compress.

18. Compress according to Claim 15, characterized in that the respective electrode (31 or 32) has the form of a comb, in that the distance between the teeth (36) of one of the combs (31) is greater than the width of a tooth (37) of the other comb (32) and in that the edges of the electrodes can be further reorganized by forming smaller teeth.

19. Compress according to Claim 1, characterized in that the material of the guiding layer (2) is filled with active substances such as with activated carbon, blood-staunching components or the like.

20. Compress according to Claim 2, characterized in that the guiding layer (2) is formed by a layer of a material which forms gel when it is moistened.

21. Method for producing the compress according to Claim 1, characterized in that a layer of material from which the guiding layer (2) is to be formed is machined for example by stamping in such a manner that it has channels (3) which extend in the direction of the surface of said layer (2), in that a surface ply (10) is heated and is placed on the top side of the guiding layer (2), where it can cool down and be joined thereby to the guiding layer, in that a layer of material which constitutes the depot layer (1) is assigned to that side of the guiding layer (2) which faces the back of the compress and in that said two layers (1 and 2) are joined to one another.

22. Method according to Claim 21, characterized in that a layer (15) of a material which is substantially liquid-tight but is capable of breathing is assigned to that side of the depot layer (1) which faces the back of the compress and in that the edges (16, 17) of the surface ply (10) and of the lower sealing ply (15) are bonded to one another, for example by means of ultrasound.

## Revendications

1. Compresse pour soigner les plaies, avec une couche de dépôt (1) pour collecter les sécrétions de la plaie, caractérisée en ce qu'une couche de guidage (2) est affectée à la face de la couche de dépôt (1) tournée vers la face avant de la compresse, c'est-à-dire est placée avant elle, en ce que cette couche de guidage est plus mince que la couche de dépôt, en ce que des canaux (3) sont réalisés dans la surface de la couche de guidage (2) qui est éloignée de la couche de dépôt (1), en ce que l'ouverture (6) de ces canaux (3) est éloignée de la couche de dépôt (1) et en ce que les canaux (3) s'étendent dans la direction de la surface de la couche de guidage (2), ceux-ci permettant aux sécrétions, qui parviennent sur la couche de guidage (2), de s'éparpiller principalement le long de cette couche (2) avant de parvenir à la couche de dépôt (1).

2. Compresse selon la revendication 1, caractérisée en ce que la couche de guidage (2) est réalisée en tant que couche de fibres comprimées, en ce que les canaux (3) sont réalisés en comprimant localement les fibres, de sorte que la densité des fibres dans la zone du fond (4) du canal (3) soit supérieure à la densité des fibres dans la zone des parois (5) du canal, en ce que l'épaisseur du fond du canal peut être égale à la profondeur du canal (3) ou inférieure à celle-ci, et en ce que la face inférieure de la couche de fibres formant le fond du canal est en contact avec la face supérieure de la couche de dépôt (1).

3. Compresse selon la revendication 1, caractérisée en ce que les canaux (3) sont ouverts en direction de la face avant de la compresse et par conséquent sont tournés du côté opposé à la couche de dépôt et en ce que les canaux (3) présentent une section transversale pratiquement en forme de U ou de V, les canaux (3) pouvant être essentiellement parallèles entre eux et présenter un tracé en forme de zig-zag.

4. Compresse selon la revendication 1, caractérisée en ce que la face avant de la couche de guidage (2) est pourvue d'une nappe superficielle (10) et en ce que cette nappe superficielle (10) est réalisée de telle sorte qu'elle soit perméable aux sécrétions et qu'elle ne puisse pas coller à la plaie.

5. Compresse selon la revendication 4, caractérisée en ce que la nappe superficielle (10) est constituée de deux couches (11, 12) perméables aux sécrétions et qui sont reliées entre elles de manière intime, en ce que la couche externe, ou couche de couverture (11) contient principalement un matériau, par exemple du polypropylène, qui n'absorbe pas les sécrétions de la plaie, en ce que la couche interne, ou couche de liaison (12), contient principalement un matériau, par exemple du polyéthylène, qui permet de réaliser une liaison entre la couche de couverture (11) et la couche de guidage (2), et en ce que la nappe superficielle (10) peut contenir des fibres qui forment un voile.

6. Compresse selon la revendication 4, caractérisée en ce que la nappe superficielle (10) est réalisée en tant que membrane de feuille d'aluminium perforée.

7. Compresse selon la revendication 1, caractérisée en ce que la face arrière de la couche de dépôt (1) est pourvue d'une couche (15) substantiellement imperméable aux liquides mais toutefois respirante et en ce que cette couche de protection (15) peut être réalisée en tant que voile de microfibres, par exemple en polyéthylène.

8. Compresse selon la revendication 7, caractérisée en ce que la couche de dépôt (1) et la couche de guidage (2) ont pratiquement les mêmes dimensions, en ce que les dimensions de la nappe superficielle (10) et de la couche terminale inférieure (15) sont légèrement supérieures aux dimensions du centre (1, 2) de la compresse, de sorte que la nappe superficielle (10) et la couche terminale (15) présentent des bords (16, 17) dépassant du centre (1, 2), en ce que la largeur de ces parties de bord (16, 17) qui dépassent est juste suffisante pour pouvoir souder les bords (16, 17) ensemble et immobiliser le centre (1, 2) dans une telle enveloppe (10, 15).

9. Compresse selon la revendication 4, caractérisée en ce que la face externe (11) de la nappe superficielle (10) est pourvue d'une couche (25) qui est perméable aux sécrétions de la plaie et qui sert d'organe de séparation entre la nappe superficielle (10) et la plaie et en ce que l'organe de séparation (25) recouvre au moins une partie de la face externe de la nappe superficielle (10).

10. Compresse selon la revendication 9, caractérisée en ce que l'organe de séparation (25) est réalisé en tant que produit semblable à un treillis, dont au moins la surface est en un matériau non mouillable, avantageusement en métal, comme par exemple de l'argent ou de l'or.

11. Compresse selon la revendication 10, caractérisée en ce que le treillis (25) est en métal ou en ce que le treillis présente un centre en matière synthétique, comme par exemple en polyester, en polyamide ou en polypropylène, dont la surface est métallisée.

12. Compresse selon la revendication 4, caractérisée en ce que le treillis (25) présente deux réseaux de lignes (26, 27), qui reposent sur la face externe de la nappe superficielle (10), en ce que ces lignes (26, 27) se trouvent pratiquement dans le même plan et se croisent en formant un angle aigü entre elles, en ce que dans la zone des intersections de ces réseaux de lignes (26, 27) se trouvent des accumulations de matière (28) adhérant à la surface, à l'aide desquelles l'intersection respective de deux lignes 26 et 27 est renforcée, et en ce que les accumulations de matière (28) peuvent présenter un contour circulaire et avoir un diamètre environ deux fois plus grand que la largeur des lignes (26, 27).

13. Compresse selon la revendication 12, caractérisée en ce que le treillis (25) contient au moins un réseau de lignes de stabilisation (30) dont la largeur est avantageusement égale à la largeur des lignes diagonales (26, 27), en ce que les lignes de stabilisation (30) se trouvent dans le même plan que les lignes diagonales (26, 27) et que les accumulations de matière (28), en ce que chaque ligne de stabilisation (30) passe par les intersections (28) situées les unes derrières les autres dans la direction longitudinale de celle-ci.

14. Compresse selon la revendication 13, caractérisée en ce qu'il est prévu un deuxième réseau de lignes de stabilisation qui courrent ou sont placées perpendiculairement aux lignes de stabilisation (30) du premier réseau.

15. Compresse selon la revendication 4, caractérisée en ce que la face externe (11) de la nappe superficielle (10) est pourvue d'au moins deux pistes conductives ou électrodes (31, 32) l'une à côté de l'autre, qui peuvent être raccordées à une source de courant et en ce que ces électrodes peuvent être des électrodes multiples et/ou à membrures.

16. Compresse selon la revendication 15, caractérisée en ce que des barrettes (33, 34) sont affectées aux électrodes (31, 32), lesquelles barrettes étant en contact électrique avec les électrodes (31, 32) et auxquelles les fils conduisant à une source de courant peuvent être raccordés.

17. Compresse selon la revendication 16, caractérisée en ce que les barrettes (33, 34) sont réalisées en tant qu'attaches en forme de U qui sont placées sur un bord de la compresse, en ce qu'entre les branches de ces attaches (33, 34) est serrée la portion de bord de la partie textile du corps de compresse et en ce que l'électrode à laquelle chaque attache est affectée, est serrée entre l'une des branches de l'attache et la partie textile de la compresse.

18. Compresse selon la revendication 15, caractérisée en ce que chaque électrode respective (31, respectivement 32) présente la forme d'un peigne, en ce que l'intervalle entre les dents (36) de l'un des peignes (31) est supérieur à la largeur d'une dent (37) de l'autre peigne (32), et en ce que les arêtes des électrodes peuvent être pourvues de membrures supplémentaires en formant de plus petites dents.

19. Compresse selon la revendication 1, caractérisée en ce que le matériau de la couche de guidage (2) est mélangé à des substances actives telles que du charbon actif, des composants coagulants ou similaire.

20. Compresse selon la revendication 2, caractérisée en ce que la couche de guidage (2) est formée par une couche en un matériau qui forme un gel lorsqu'il est mouillé.

21. Procédé de fabrication de la compresse selon la revendication 1, caractérisé en ce qu'une couche de matériau, qui doit fournir la couche de guidage (2), par exemple par estampage est traitée de telle sorte qu'elle présente des canaux (3), qui s'étendent dans la direction de la surface de cette couche (2), en ce qu'une nappe superficielle (10) est chauffée et appliquée à la face supérieure de la couche de guidage (2), où elle peut refroidir et se lier ainsi à la couche de guidage, en ce qu'une couche de matériau, qui représente la couche de dépôt (1), est affectée à la face de la couche de guidage (2) qui est tournée vers la face arrière de la compresse, et en ce que ces deux couches (1 et 2) sont liées ensemble.

22. Procédé selon la revendication 21, caractérisé en ce qu'une couche (15) en un matériau essentiellement imperméable aux liquides mais cependant respirant est affectée à la face de la couche de dépôt (1) qui est tournée vers la face arrière de la compresse, et en ce que les bords (16, 17) de la nappe superficielle (10) et de la nappe terminale inférieure (15) sont soudés ensemble, par exemple par ultrasons.
